# EUROPEAN PATENT APPLICATION

(11) **EP 3 575 812 A1**
(43) Date of publication of application: **04.12.2019**
(21) Application number: 18174524.1
(22) Date of filing: 28.05.2018
(51) Int. Cl.: G01R 33/28, G01R 33/54

(54) **A METHOD, COMPUTER PROGRAM PRODUCT AND DEVICE FOR CLASSIFYING SOUND IN MRI AND FOR TRAINING A PATIENT**

(71) Applicant: Koninklijke Philips N.V., 5656 AE Eindhoven (NL)
(72) Inventor: AMTHOR, Thomas Erik, 5656 AE Eindhoven (NL); HEUVELINK-MARCK, Annerieke, 5656 AE Eindhoven (NL); DOTSCH, Ron, 5656 AE Eindhoven (NL); NAUTS, Sanne, 5656 AE Eindhoven (NL); SAINI, Privender Kaur, 5656 AE Eindhoven (NL); TASAR, Ozgur, 5656 AE Eindhoven (NL)
(74) Representative: de Haan, Poul Erik

(57) **Abstract**

It is an object of the invention to increase the predictability of the MRI exam for the patients. This object is achieved by a method for classifying sound of a magnetic resonance imaging sequence into a sound category, wherein the magnetic resonance sequence comprises a one or more sound blocks, wherein individual sound blocks have signal characteristics and wherein sound blocks having similar characteristics are to be classified into the same sound category, the method comprising the steps of:
- receiving information about one or more gradient waveforms to be used in the magnetic resonance imaging sequence and;
- using a classification algorithm to map the waveform information to a sound category and;
- allocating a visual to the sound category.

## Description

### FIELD OF THE INVENTION

The invention relates to the field of magnetic resonance imaging

### BACKGROUND OF THE INVENTION

During magnetic resonance imaging (MRI), acoustic noises are produced by the gradient coil. As a result, patients may feel uncomfortable during MRI examinations. As such, many adults feel nervous when undergoing an MRI-exam, and children often receive sedation or general anesthesia before a scan. Anesthesia/sedation at a young age may have long-term negative health effects. Moreover, an exam with sedation is approximately three times as expensive as an awake exam and an exam with anesthesia is nine times as expensive as an awake exam.

US 2013/0275086 A1 discloses a method and magnetic resonance apparatus to determine and/or adjust a noise volume for a magnetic resonance examination, a selection of a magnetic resonance sequence for the magnetic resonance examination of a subject to be examined hereby is made and an automatic calculation of an expected noise volume for the magnetic resonance examination is made in a volume determination unit using protocol parameters of the selected magnetic resonance sequence. Information about the expected volume is provided to an operator via a user interface.

### SUMMARY OF THE INVENTION

During MRI, patients are exposed to very loud and unfamiliar acoustic noises. These noises can be very different in sound pressure and spectral features. It is an insight of the inventors that the unexpected acoustic noises may increase the anxiety in patients. It is a further insight of the inventors that increasing the predictability of a MRI exam may reduce anxiety in some patients. Reduced anxiety may result in a reduced need for anesthesia or sedation in a selected group of (young) patients. Also, reduced anxiety may result in improved image quality in some patients, as a result of reduced motion artefacts.

It is an object of the invention to increase the predictability of the MRI exam for the patients. This object is achieved by the independent method, computer program products, MRI system and device claims.

One of the insights underlying embodiments of the invention is that predictablility may be increased if a patient whose MRI data is being acquired knows upfront what kind of sounds will be produced by the MRI system. This can for example be achieved by providing a real-time visualization of the type of sound to be expected during the next period, e.g. in the order of seconds. Different visuals or visual elements will be allocated to different sound categories. After a certain image acquisition period, the patient will have learned to associate the visuals with their corresponding sound categories. Seeing the visuals representing the upcoming sounds will then increase the predictability of the (remaining) MRI exam for the patient.

Also, the predictability for the patient may increase when the patient is presented with specific MRI sounds prior to the actual MRI data acquisition during a here called "learning" or "training phase". In this way, the patient is already familiar with the sounds of of the MRI system, which in turn may reduce anxiety in some patients. Even further, these two aspects may be combined. The learning phase may be used to learn to associate certain sounds with certain visuals or visual elements. In this way, the patient is acquainted with the visuals and the corresponding sounds at the moment the MRI data acquisition starts. By seeing the visuals representing the upcoming sounds, the predictability of the MRI exam is increased for the patient.

Because most sounds are produced by the gradient system, the above can be achieved by classifying parts of a waveform to be send to a gradient system of the MRI system. The purpose of this classification is to group parts of the waveform together to form blocks that correspond to the acoustic noise "elements" identified by the human ear and to sort these blocks into categories of different types of sound. These acoustic noise elements are herein defined as sound categories. A temporally connected block of the same sound category is herein called a sound block. Example of sound categories are:
- click sound
- hammering sound
- long chirp
- monotonous humming

But other examples may be possible. Further, also a full MRI sequence could be considered a sound block.

The invention has a plurality of aspects that all share the same inventive concept. However, embodiments of the invention are not necessarily covered within a single method, computer program product and / or device. Preferably, parts of the invention are performed by separate computer program products and / or devices.

According to one aspect, the invention is a method for classifying sound of a MRI sequence into a sound category, wherein the magnetic resonance sequence comprises one or more sound blocks, wherein individual sound blocks have signal characteristics and wherein sound blocks having similar characteristics are to be classified into the same sound category, the method comprising the steps of:
- receiving information about one or more gradient waveforms to be used in the MRI sequence. This information could for example be the gradient waveforms themselves, but also the resulting sound, which in general will have a similar waveform as the gradient waveform. Also, the information could be the MRI sequence, as the MRI sequence also provides for information about the gradient waveforms that will be used when using that MRI sequence.
- using a classification algorithm to map waveform information to a sound category and;
- allocating a visual to the sound category.

The sound blocks could be a complete MRI sequence, but preferably a single MRI sequence comprises a plurality of (temporally connected) sound blocks. The sound blocks can be identified by the characteristics of their frequency spectra.

This method can be performed by the MRI system, e.g. real-time as will be discussed in the detailed description. Alternatively or additionally, the method can be performed prior to the magnetic resonance, for example on the MRI system, on an independent workstation or in the cloud.

According to further embodiments of the invention, the method also comprises the step of displaying the visual, preferably to a patient.

According to further embodiments the method comprises the step of
- acquiring MRI data by means of the MRI sequence by means of a MRI system,
wherein the visual corresponding to a certain sound block is displayed prior to the acoustic display of that sound block by the MRI system.

Seeing the visuals corresponding to a sound category may increase the predictability of the MRI exam for the patient.

According to further embodiments of the invention one or more gradient waveforms to be used in the MRI sequence are received from the MRI system in real-time. This embodiment is advantageous, because it provides for flexibility in unexpected situations. For example when an exam needs to be paused, stopped or in any way altered, still the correct visuals will be displayed prior to the generation of the sound blocks by the MRI system.

According to further aspects, the invention is a computer program product configured for classifying sound, wherein the computer program product comprises program code means for causing a computer to carry out the steps of a method of according to any of claims the above.

According to further aspects, the invention is an MRI system, wherein the MRI system is configured for acquiring MRI data using an MRI sequence, wherein the magnetic resonance sequence comprises one or a plurality of sound blocks, wherein individual sound blocks have signal characteristics and wherein sound blocks having similar characteristics are classified into the same sound category and wherein a different visual is allocated to each individual sound category, wherein the MRI system comprises
- a gradient system configured for producing magnetic field gradients, wherein the use of the gradient system results in production of sound blocks and
- a data storage comprising a plurality of sound categories and visuals allocated to each of the sound categories and;
- a display or display means configured for displaying a visual corresponding to the sound category of a sound block within a time interval before the MRI system produces the sound block as a result of the use of the gradient system.

Preferably, the visual is displayed at least 60 seconds before the sound block is produced by the MRI system. The visual can be displayed in many different ways, which to a very large extent are known in the art. For example the visual can be displayed on a display or by using (virtual reality) glasses, but it could also be projected in such a way that it is visible to the patient, e.g. on the wall of the examination room, such that it can be viewed by the patient, while being in the MRI system, e.g. by using a mirror. Also the visual may be projected in the bore of the MRI system.

According to further embodiments of the invention the MRI system further comprises
- a module for monitoring gradient waveforms about to be sent to the gradient system and
- a classification module configured for mapping waveform information to a sound category.

This embodiment is advantageous, because by monitoring the gradient waveforms in real time, the embodiment provides for flexibility in unexpected situations. For example when an exam needs to be paused, stopped or in any way altered, still the correct visuals will be displayed prior to the generation of the sound blocks by the MRI system.

According to another embodiment, the MRI system comprises a storage with a visual rendering for one or more of the MRI sequences, wherein the visual rendering comprise one or more of the visuals, wherein the MRI system is configured to synchronize the visual rendering with a selected MRI sequence and display the visual rendering during MRI acquisition by means of the selected MRI sequence.

According to further aspects of the invention, the invention is a method for training a patient in associating different visuals with different sound categories in order to increase the predictability of a future MRI exam for the patient, wherein the MRI exam comprises one or more magnetic resonance sequences, wherein the one or more magnetic resonance sequences comprise one or a plurality of sound blocks, wherein individual sound blocks have signal characteristics and wherein a different visual is allocated to each individual sound category, wherein a similar combination of sound categories and visuals is planned to be used in the future magnetic resonance exam of the patient, wherein the method comprises the steps of
- receiving data comprising a plurality of sound categories and visuals allocated to each of the sound categories and;
- providing to the patient simultaneously or within a time interval of less than 60 seconds a sound from a sound category and a visual allocated to the sound category.

More preferably the time interval is less than 50 seconds, even more preferably it is less than 30 seconds, even more preferably it is less than 20 seconds, even more preferably it is less than 10 seconds, even more preferably it is less than 10 seconds, even more preferably it is less than 5 or less than 1 second. The time interval should at least be long enough for a user to register the visual and it should be short enough to associate the visual with the sound category.

This aspect is advantageous, because by means of the method a patient may already become acquainted to the MRI specific sounds, which may increase the predictability of the magnetic resonance exam and may therefore reduce the anxiety in some patients. Further, by learning the patients to associate certain visuals with specific sounds or sound categories, the predictability of the MRI exam may be further increased when the same visuals are used to notify the patient for the upcoming sounds. This can for example be achieved by means of a movie, wherein the visuals are displayed while a sound from the sound category to which it is allocated is played simultaneously or within a certain time interval. Alternatively or additionally, this aspect can be implemented in the form of a game. Examples of such games are described below.

According to embodiments of the method further comprises the steps of
- providing a plurality of the visuals to the patient and;
- receiving a user input from the patient, wherein the user input comprises a selection of a visual from the plurality of visuals and;
- in response to the user input providing a sound to the patient, wherein the sound is a sound from the sound category to which the selected visual is allocated.

This embodiment may be advantageous, because it is more interactive and may therefore be more entertaining for the (pediatric) patient. This may result in a better engagement in the training and therefore in an improved training result. For example the patient could be provided with a plurality of buttons displaying the visuals. The patient may use the buttons to play music, either based on his own creativity or by replaying a song or following instructions. As will be clear to those skilled in the art, such buttons could be used in many different kinds of games. The buttons could either be real buttons or buttons in a computer program product, like e.g. an app.

According to further aspects, the invention is a computer program product configured for training a patient, wherein the computer program product comprises program code means for causing a computer to carry out the steps of a method of according to the above.

According to further aspects the invention is a device for training a patient in associating different visuals with different sound categories in order to increase the predictability of a future MRI exam for the patient, wherein the MRI exam comprises one or more magnetic resonance sequences, wherein the one or more magnetic resonance sequences comprise one or a plurality of sound blocks, wherein individual sound blocks have specific signal characteristics and wherein a different visual is allocated to each individual sound category, wherein a similar combination of sound categories and visuals is planned to be used in the future magnetic resonance exam of the patient, wherein the device comprises:
- a plurality of input receiving means configured for receiving an input from a patient, wherein each of the input receiving means display one of the different visuals and;
- a sound producing module configured for producing a sound in response to user input received from an input receiving means selected by a user, wherein the produced sound is a sound in the sound category corresponding to the visual displayed on the user selected input receiving means.

The device could for example be an (educational) toy. This embodiment is advantageous, because it may stimulate (young) patients to engage in the training. The sound producing module could for example comprise a speaker.

According to embodiments of the invention, the size of the visuals in one or more directions in varied based on at least any of the following parameters: duration of the sound block, intensity of the sound, center frequency. This embodiment is advantageous, because it provides for more information about the upcoming sound. This may further increase the predictability of the MRI exam for the patient.

These and other aspects of the invention will be apparent from and elucidated with reference to the embodiments described hereinafter.

### BRIEF DESCRIPTION OF THE FIGURES

Fig. 1 diagrammatically shows a basic setup of a system according to embodiments of the invention and;
Fig. 2 diagrammatically shows a processing procedure according to embodiments of the invention and;
Fig. 3 shows two examples of potential visualizations
Fig.4 diagrammatically shows systems according to other embodiments of the invention and;
Fig. 5 shows a possible scanning phase system architecture for an implementation of embodiments of the invention and;
Figs. 6 diagrammitically shows two examples of products that can be used in the training phase.

### DETAILED DESCRIPTION OF THE INVENTION

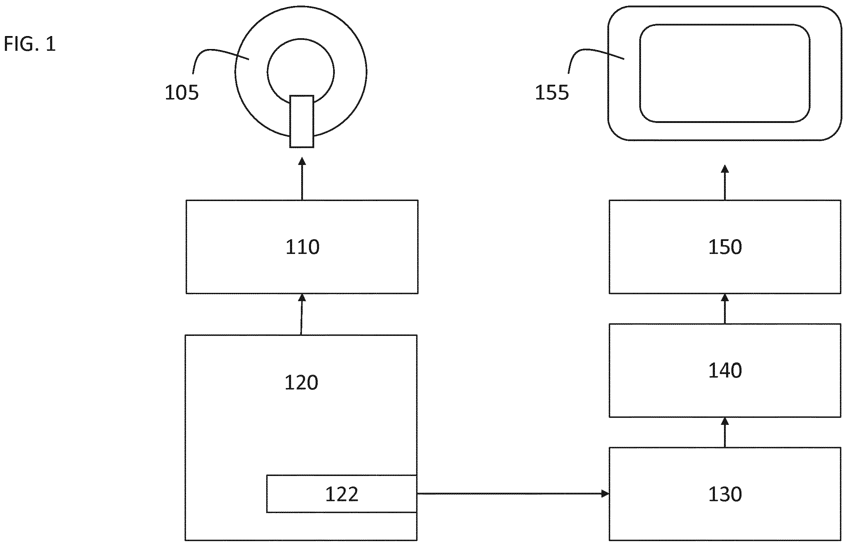

Fig. 1 diagrammatically shows a basic setup of a system according to embodiments of the invention. The system comprises an MRI system control unit 120. The MRI system control unit (scanner host) calculates the gradient wave forms according to a selected MRI sequence. These waveforms are sent to the gradient amplifier 110, and the resulting gradient currents are introduced to the gradient coils, producing different types of sounds. The gradient system is part of a MRI system 105. This part of the setup represents the currently normal implementation of an magnetic resonance gradient control. Embodiments of the invention proposes to extend the scanner host with an interface 122 to access the calculated (future) gradient waveforms and their (exact) timing, including the (absolute) time when the waveforms will be applied. The interface 122 is accessible during scanner operation and will deliver information about the currently expected future waveforms. If a scan is aborted or scan parameters are changed before starting a scan, the information about the expected gradient waveforms is updated in real time.

The information about the gradient waveforms is first processed by a prediction module 130. The prediction module is configured for mapping waveform information to a sound. This could for example be achieved by means of an algorithm containing a magneto-mechanical model of the gradient coil (and possibly its surrounding elements) to calculate the mechanical response to the gradient wave forms. This mechanical response can be used as a prediction of the audible sound. Calculation of the mechanical response could be performed as a function of frequency and amplitude for each of the three gradient directions separately. The complete response of the MRI sequence can then be simulated by a linear superposition of responses for all involved frequencies. The system can be modeled using coupled magnetomechanical differential equations, which may be solved with available Multiphysics toolboxes. Examples for commercial Multiphysics products are ANSYS and COMSOL.

The information about the audible sound is then processed by a classification module 140. The purpose of this classification is to group parts of the waveform together to form blocks that correspond to the sound categories identified by the human ear and to sort these blocks into categories of different types of sound.
Example for noise block categories are:
- click sound
- hammering sound
- long chirp
- monotonous humming
- etc

Classification can be done either by mapping of known features, such as power and center frequency, to a previously defined set of categories, or by using machine learning to perform this mapping based on a test data set of sound blocks labelled by humans. After defining or creating the sound categories a different visual will be allocated to each of the different sound categories.

It should be noted that some types of algorithms, e.g. algorithms based on artificial intelligence, may be capable of directly classifying a waveform information into a sound category without the need for a separate prediction module 130.

The sound classification algorithm could comprise two steps:
1. Identification of distinct blocks, possibly separated by silence (sound blocks) and
2. Classification of the individual sound blocks.

Also, the algorithm could be more simple. The algorithm could for example directly assign a visual to a type of sequence, e.g. a square to a T1w sequence and a triangle to a FLAIR sequence.

In addition to the sound category, each sound block could be assigned a sound intensity value and the absolute timestamps of start and end of the block. A sound block could for example be described by the following data set: start time, end time (alternatively: duration), sound category and optionally, sound parameters (e.g., intensity, center frequency)

Using the expected and previous sound blocks (e.g., covering a time span of +/- 10, 20, 30, 40, 50 or 60 seconds relative to the current time), a visualization engine 150 translates the blocks and their properties to visual objects (visuals) to be presented to the patient on a display device 155. The visualization may also include a marker representing the current time. The visualization may be updated in real time, i.e. the visual objects move across the screen, showing the patient which sound blocks are to be expected and when to expect them.

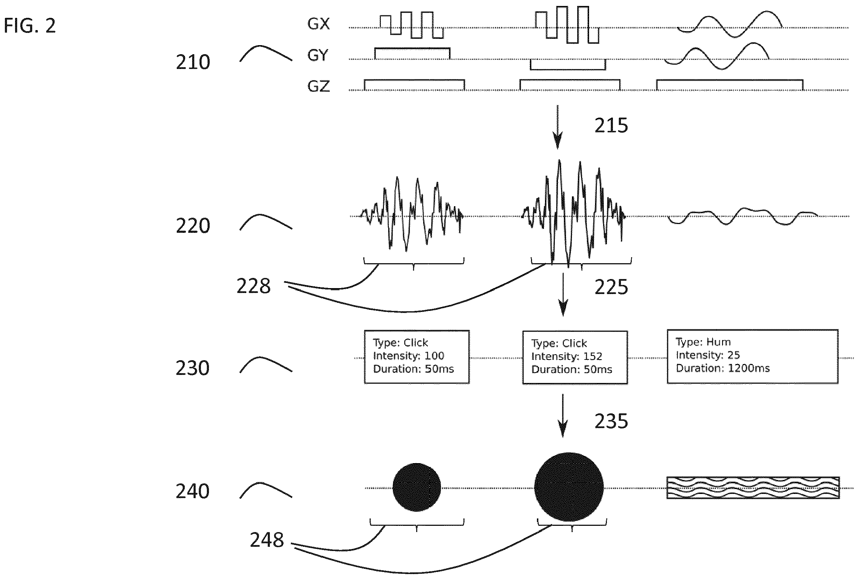

Fig. 2 diagrammatically shows a processing procedure according to embodiments of the invention. In this example, the MRI sequence 210 produces clicking and humming sounds 220. These sounds 215 can be predicted by means of the prediction module 130. These sound can be identified as sound blocks 228. The sound blocks can be classified 225 to a sound category 230 and then transformed 235 into visualization objects or visuals 248 allocated to the sound category.

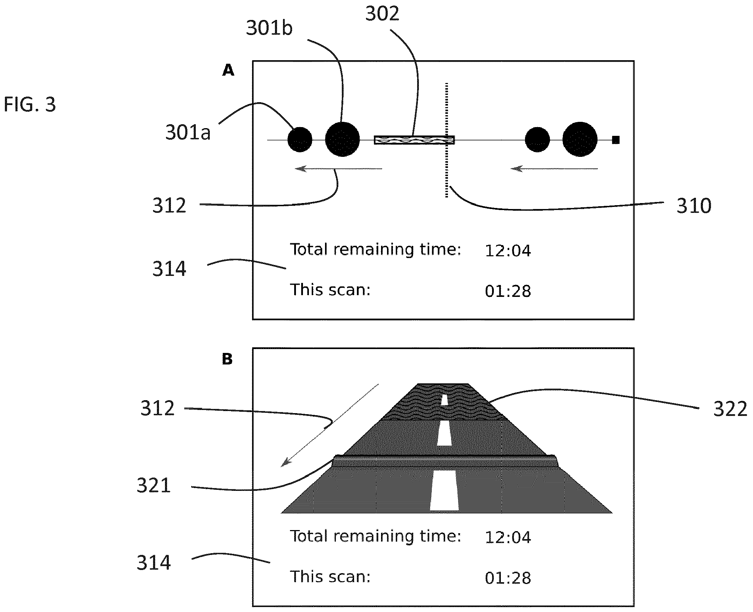

The visualization 240 can have many different shapes. Figure 3 shows two examples of potential visualizations. Figure 3A shows a moving timeline visualization where sound blocks 301a, 301b, 302 are visualized as symbols moving from right to left. Arrow 312 indicates the direction of movement. Vertical line 310 indicates the current moment, so that the symbol currently crossing the line corresponds to the sound audible in the same moment. The type and intensity (or other properties) of the sound block is encoded in the shape, size, and fill style of the symbol. For example sound block 301a and 301b belong to the same sound category, but their sizes differ. The difference in size could be used to indicate for example that the intensity of the sound of sound block 301b is larger than the intensity of 301a. In addition to the sound timeline, other information 314 can be displayed, such as the remaining scan time.

Fig. 3B shows another possible visualization of sound blocks 321, 322. The sound blocks are mapped to visual objects (visuals) in a way reminding of computer games. In this example, time is represented by driving along a road. The arrow 312 indicates the direction of motion of the visualization. The current moment is indicated by the lower edge (or alternatively, a car on the road). Sound blocks appear in the form of speed bumps, different road surfaces etc. The properties of the noise blocks (such as intensity) can further be encoded in the size of these objects, their colors, or their visual appearance. Such a visualization not only lets the patient expect the noise, it may also make the noises appear more natural, because they are connected to known visual scenes.

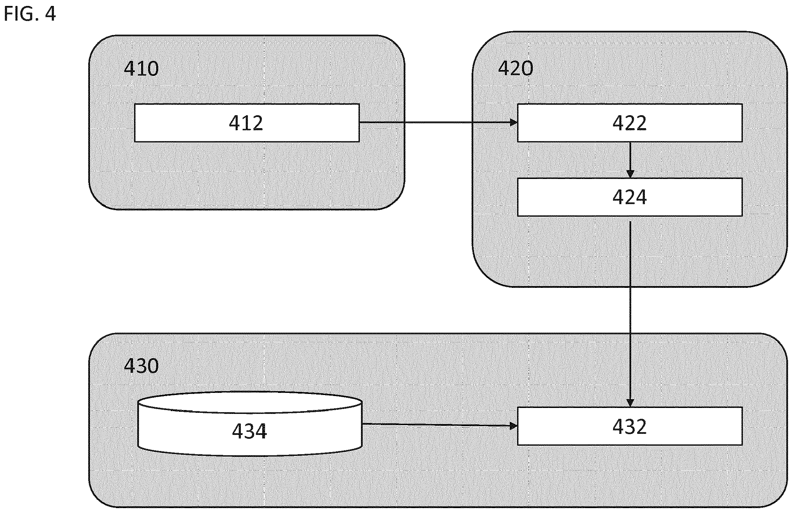

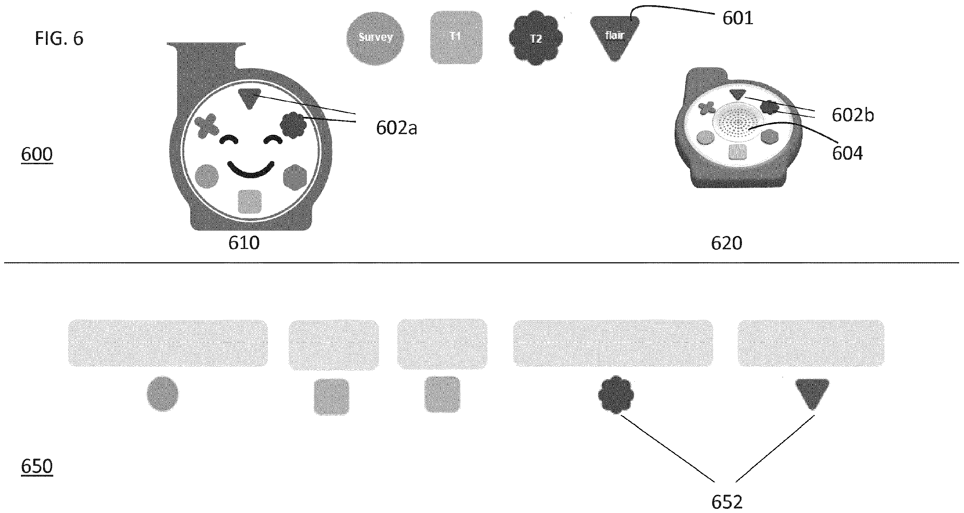

Fig. 4 diagrammatically shows systems according to other embodiments of the invention. Typically, hospitals have scanning protocols for different anatomies and clinical questions. Depending on the scanning protocol that is ordered for an MRI exam, a patient will be exposed to different MRI sequences, and, thus, to different sound categories. According to embodiments of the invention, when an MRI exam is ordered, a healthcare provider (e.g., a childlife specialist or referring physician) selects or enters the planned protocol in a clinician dashboard 410 by means of a protocol selector 412. The dashboard is connected to a processing unit 420 that receives as input the selected protocols and converts those to pulse sequences based on a database with protocol and associated pulse sequences (not shown in Fig. 4). Subsequently, an algorithm clusters all pulses that are part of all selected pulse sequences together based on their auditory similarity 422, and then ranks the corresponding sound categories on the basis of their frequency of occurrence in the planned session 424. The top N most occurring sound categories are transmitted to a patient-facing app/toy (430 and Fig. 6 600), where N preferably equals the number of symbol buttons Fig. 6, 602 available to the patient to press on the app/toy 430. The app/toy displays for each selected sound category the associated symbol and sound 432 and then presents the appropriate sound when one of the symbol buttons is pressed. The associations between symbol and pulses are later re-used in the scanning phase.The sound categories and their allocated visuals are stored in a storage 434 in the app / toy 430.

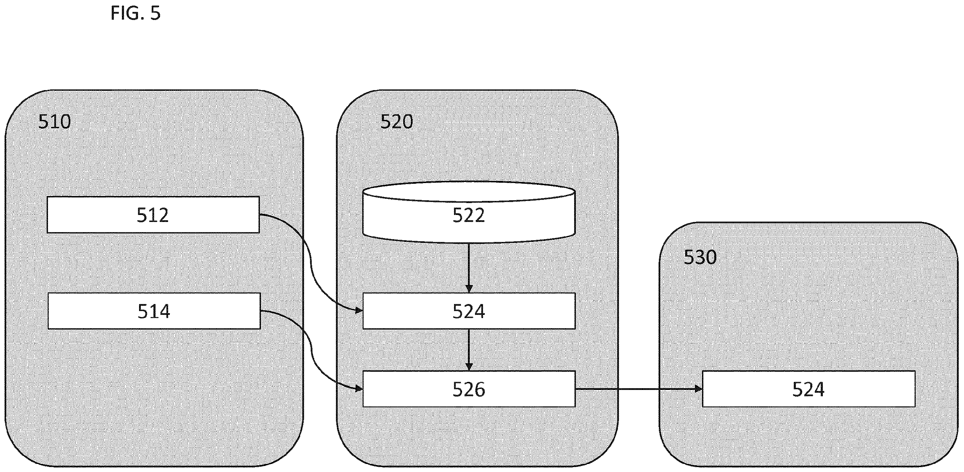

After the learning phase comes the scanning phase, during which patients are in the bore of the MRI-scanner. During this phase, patients receive real-time feedback about the sequence that is currently being performed. Additionally, they may be presented with information about the sequences that will be performed after this. Figure 5 shows a possible scanning phase system architecture for an implementation of embodiments of the invention. The system comprises an MRI tech console 510, a processing unit 520 and a (in bore) display or display means 530.

Before the scanning phase commences, the MR technologist sets up the session and schedules the relevant pulse sequences 512. The system 520 imports the scheduled pulse sequences into a processing unit 520 that consists of three elements:
1. A database 522 that stores assets (visuals e.g., images like symbols and/or animations, depending on embodiment) and their associations with specific pulses (the same associations as in the database of the learning phase);
2. A buffer 524 which is filled with visual renderings of the planned pulse sequences, ready to be transferred to the in-bore display when needed; and
3. A selector mechanism 526 that selects which rendering in the buffer to transfer to the (in-bore) display depending on which pulse sequence the MRI technician decides to execute.

Once the scheduled pulse sequences are loaded into the processing unit, each pulse sequence is automatically converted into visual renderings of the sequence based on the specific pulses that are presented in the sequence, their temporal order, their timing and possibly other properties such as loudness, pitch or subjective discomfort. These visual renderings are synchronized with the MRI sequence. The visual renderings of the time pulses are stored in a session buffer, along with an id that matches them to a specific sequence.

When the scanning phase starts, the MR technologist initiates the first pulse sequence in the MRI tech console 512. This is communicated in advance to the processing unit 520. The processing unit in turn selects the visual rendering associated with the pulse sequence from the buffer 524 and transfers it to the unit controlling the (in-bore) display, which in turn displays the visual renderings 524 at the moment the pulse sequence starts.

After the sequence was completed, the MRI technologist may decide either to continue with the next planned pulse sequence, or to repeat a previous sequence 514 (for instance because there was excessive movement during the scan, resulting in reduced image quality). The next sequence (either planned or any previous sequence) is then communicated to the processing unit, just before it is initiated. The same logic as described above is repeated for every pulse sequence, until all planned sequences and unplanned repetitions have been completed and the session is terminated by the MR technologist.

Fig. 6 diagrammatically shows two examples of products that can be used in the training phase 600, a software application 610 and a physical device 620. In some embodiments, during the learning phase, a game is used to teach patients the relationship 601 between MRI-sounds and visuals. The sound blocks could be a complete MRI sequence, but preferably these sound blocks are groups of waveforms that correspond to the sound categories identified by the human ear. The sound blocks can be identified by the characteristics of their frequency spectra.

The visuals used 602a, 602b can serve as buttons, and a user may create music by pressing these buttons and combining sounds, akin to a children's musical toy. The physical device (toy) also comprises a sound producing module 604 e.g. a speaker in order to produce the sound. Further, the physical device comprises a data storage comprising the sound categories or sound representative for the sound categories and their link to the visuals allocated to them. The device may be made such that new data can be easily uploaded to the device in order to adapt the device to different MRI exams and their respective sounds. The software application or physical device 620 can be made available in the waiting room of a hospital or can be provided to the patient to be used at home. Whenever a (pediatric) patient presses a button on the musical toy, the accompanying MRI-sound is generated. Instead of using arbitrary symbols, the symbols may also be linked to the frequency and/or pitch of the sound. For example, the sound categories can be associated with animations of a character jumping rope in the same frequency as the sounds in the sound category.

Fig. 6 in addition shows the scanning phase 650. The same visuals 652 are used during scanning as were used during the training phase.

Also, the software application 610 or physical device 620 could be used to display a movie, wherein the visuals in the movie are displayed while a sound from the sound category to which it is allocated is played simultaneously or within a certain time interval.

The relationship between visuals and sound blocks used for a specific patient may be stored. In this way the same visual / sound block combination could be used when a patient comes for a rescan or follow up.

Whilst the invention has been illustrated and described in detail in the drawings and foregoing description, such illustrations and description are to be considered illustrative or exemplary and not restrictive; the invention is not limited to the disclosed embodiments.

## Claims

1. A method for classifying sound of a magnetic resonance imaging sequence into a sound category, wherein the magnetic resonance sequence comprises a one or more sound blocks, wherein individual sound blocks have signal characteristics and wherein sound blocks having similar characteristics are to be classified into the same sound category, the method comprising the steps of:
- receiving information about one or more gradient waveforms to be used in the magnetic resonance imaging sequence and;
- using a classification algorithm to map the waveform information to a sound category and;
- allocating a visual to the sound category.

2. A method according to claim 1, further comprising the step of displaying the visual.

3. A method according to claim 2, further comprising the step of:
- acquiring magnetic resonance imaging data by means of the magnetic resonance imaging sequence by means of a magnetic resonance imaging system,
- wherein the visual corresponding to a certain sound block is displayed prior to the acoustic display of that sound block by the magnetic resonance imaging system.

4. A method according to claim 3, wherein one or more gradient waveforms to be used in the magnetic resonance imaging sequence are received from the magnetic resonance imaging system in real-time.

5. Computer program product configured for classifying sound, wherein the computer program product comprises program code means for causing a computer to carry out the steps of a method of according to any of claims 1-4.

6. A magnetic resonance imaging system, wherein the magnetic resonance imaging system is configured for acquiring magnetic resonance imaging data using a magnetic resonance imaging sequence, wherein the magnetic resonance sequence comprises one or more sound blocks, wherein individual sound blocks have signal characteristics and wherein sound blocks having similar characteristics are classified into the same sound category and wherein a different visual is allocated to each individual sound category, wherein the magnetic resonance imaging system comprises
- a gradient system configured for producing magnetic field gradients, wherein the use of the gradient system results in production of sound blocks and
- a data storage comprising a plurality of sound categories and visuals allocated to each of the sound categories and;
- a display or display means configured for displaying a visual corresponding to the sound category of a sound block within a time interval before the magnetic resonance imaging system produces the sound block as a result of the use of the gradient system.

7. A magnetic resonance imaging system according to claim 6, wherein the magnetic resonance imaging system further comprises
- a module for monitoring gradient waveforms about to be sent to the gradient system and
- a classification module configured for mapping waveform information to a sound category.

8. A magnetic resonance imaging system according to claim 6, further comprising a storage with a visual rendering for one or more of the magnetic resonance imaging sequences, wherein the visual rendering comprise one or more of the visuals, wherein the magnetic resonance imaging system is configured to synchronize the visual rendering with a selected magnetic resonance imaging sequence and display the visual rendering during magnetic resonance imaging acquisition by means of the selected magnetic resonance imaging sequence.

9. A method for training a patient in associating different visuals with different sound categories in order to increase the predictability of a future magnetic resonance imaging exam for the patient, wherein the magnetic resonance imaging exam comprises one or more magnetic resonance sequences, wherein the one or more magnetic resonance sequences comprise one or more sound blocks, wherein individual sound blocks have signal characteristics and wherein a different visual is allocated to each individual sound category, wherein a similar combination of sound categories and visuals is planned to be used in the future magnetic resonance exam of the patient, wherein the method comprises the steps of
- receiving data comprising a plurality of sound categories and visuals allocated to each of the sound categories and;
- providing to the patient simultaneously or within a time interval of less than 60 seconds a sound from a sound category and a visual allocated to the sound category.

10. A method for training a patient according to claim 9, the method further comprising the steps of
- providing a plurality of the visuals to the patient and;
- receiving a user input from the patient, wherein the user input comprises a selection of a visual from the plurality of visuals and;
- in response to the user input providing a sound to the patient, wherein the sound is a sound from the sound category to which the selected visual is allocated.

11. Computer program product configured for training a patient, wherein the computer program product comprises program code means for causing a computer to carry out the steps of a method of according to any of claims 9-10.

12. A device for training a patient in associating different visuals with different sound categories in order to increase the predictability of a future magnetic resonance imaging exam for the patient, wherein the magnetic resonance imaging exam comprises one or more magnetic resonance sequences, wherein the one or more magnetic resonance sequences comprise one or more sound blocks, wherein individual sound blocks have specific signal characteristics and wherein a different visual is allocated to each individual sound category, wherein a similar combination of sound categories and visuals is planned to be used in the future magnetic resonance exam of the patient, wherein the device comprises:
- a plurality of input receiving means configured for receiving an input from a patient, wherein each of the input receiving means display one of the different visuals and;
- a sound producing module configured for producing a sound in response to user input received from an input receiving means selected by a user, wherein the produced sound is a sound in the sound category corresponding to the visual displayed on the user selected input receiving means
- a data storage comprising the sound categories or sound representative for the sound categories, wherein the data storage further provides for a link between the sound categories and the visuals allocated to them.

13. A method, computer program product, magnetic resonance imaging system or device according to any of the preceding claims, wherein the size of the visuals in one or more directions in varied based on at least any of the following parameters: duration of the sound block, intensity of the sound, center frequency.
